# EUROPEAN PATENT SPECIFICATION

(11) **EP 2 037 989 B1**
(45) Date of publication and mention of the grant of the patent: **15.09.2010**
(21) Application number: 07796586.1
(22) Date of filing: 29.06.2007
(51) Int. Cl.: A61M 5/32

(54) **MAGNETICALLY INDUCED SAFETY TECHNOLOGY**
MAGNETISCH INDUZIERTE SICHERHEITSTECHNOLOGIE
TECHNOLOGIE DE SÉCURITÉ INDUITE MAGNÉTIQUEMENT

(30) Priority: 10.07.2006 US 456248
(43) Date of publication of application: 25.03.2009
(73) Proprietor: Becton, Dickinson & Company, Franklin Lakes, NJ 07417-1880 (US)
(72) Inventor: ISAACSON, Ray, S., Roy, UT 84067 (US); CRAWFORD, Mark, Sandy, UT 84904 (US)
(74) Representative: von Kreisler Selting Werner
(86) International application number: PCT/US2007/015153
(87) International publication number: WO 2008/008206

(56) References cited:
- EP-A- 0 645 159
- EP-A- 1 568 393
- WO-A-2006/062983
- US-A1- 2005 182 362

## Description

### FIELD

This application relates to safety medical devices, in particular, to devices that secure needle tips within the devices.

### BACKGROUND

Dangers associated with accidental needle sticks are well-known. Safe handling of used needles is important to minimize transmission of harmful contaminants or infectious diseases. Many safety products today use binding as a method of securing a needle tip in a safety device to reduce the occurrence of accidental needle sticks. One way to secure a needle tip is to use a binding component that bears down on the cannula when pressure is applied off-center from the axis of the needle. This pressure forces an aperture in the binding component to bite down on the cannula, preventing the binding component from translating. When this occurs, the needle can be held in one position, generally within the confines of a housing, and accidental needle sticks can be avoided.

In some examples, the binding component contains a feature that stores mechanical energy (e.g., the coil springs of U.S. Patent Nos. 6,719,737 and 6,695,819 and the leaf spring of U.S. Patent Publication No. 2005/0182362) that bears down on the cannula and locks it in place. Other examples use frictional- based binding. Some components, however, have limited shelf lives due to, for example, relaxation of the springs. Other components are cumbersome and require relatively large housing components. In some instances, the stored mechanical devices are relatively slow in acting to bind the needle tips.

There is a need, therefore, for binding components that are reduced in size, are faster acting, and have longer shelf-lives.

EP 1 568 393 describes a safety needle device having a housing, whereby the needle can be retraced into the housing so that the needle tip is securely located within the housing. An opening of the housing is automatically closed by shielding members. These shielding members are designed as magnetic elements.

WO 2006/062983 discloses also a housing to securely locate a needle tip, whereby the whole cannula is pivoted so that the needle tip is not located inline with an opening of the housing. The rotation is caused by a biasing element, whereby this biasing element can be a magnetic biasing element.

### SUMMARY

In one aspect of the present invention, safety needle assemblies are provided that comprise a cannula comprising a proximal end and a distal end; a hub disposed on the proximal end of the cannula; a housing partially surrounding a portion of the cannula, the housing comprising a distal end; and a magnetic latch that is operative against the cannula to secure the distal end of the cannula within the housing and a binding component, which rotates due to a magnetic force, whereby edges of an aperture of said binding component bite down on or bind the cannula, when the needle tip of the cannula is retracted into the safety housing.

In another aspect of the present invention, a method of making a safety needfe assembly is provided, the method comprising: providing a cannula comprising a proximal end and a distal end; disposing a hub on the proximal end of the cannula; partially surrounding a portion of the cannula with a housing comprising a distal end; and locating a magnetic latch such that it is operative against the cannula by a binding component which rotates due to a magnetic force, whereby edges of the aperture are binding components to bite down or bind the cannula, when the needle tip of the cannula is retracted into the safety housing.

In a further aspect of the present invention, a method of securing a needle tip is provided, the method comprising: moving a safety device from a proximal end of a cannula towards the needle tip located at a distal end, wherein the safety device comprises a housing and a magnetic latch; moving the end sense member past the needle tip toward the distal end of the cannula; and rotating the binding component with magnetic force, thereby securing the needle tip within the housing whereby edges of an aperture of that binding component bite down on or bind the cannula.

### BRIEF DESCRIPTION OF THE DRAWINGS

Figs. 1 and 2 show needle assemblies according to one aspect of the invention.
Fig. 3 shows a cross-section view of a housing of a needle assembly according to one aspect of the invention.
Figs. 4-8 show a schematic view of the interior area of a housing according to an aspect of the invention.
Figs. 9-11 show a schematic view of the interior area of a housing according to an aspect of the invention.

### DETAILED DESCRIPTION

Safety medical devices, in particular, devices that secure needle tips in the devices using magnetic forces are provided. In some embodiments, the use of magnetic forces to bind needles can be faster-acting and more reliable than the use of stored mechanical energy. In other embodiments, safety devices using magnetic forces, which are not subject to components that experience mechanical relaxation, generally have longer shelf-lives than devices using stored mechanical energy. Moreover, in some embodiments, the use of magnetic latches permits safety device housings to be smaller, for example, shorter in axial length and smaller in diameter size, than devices using coils, for example. Reference to magnetic latches includes systems that use magnetically activated components, that is, components of magnetic latches move due to magnetic forces.

Reference to a magnetic material is meant to include a material that produces a magnetic field external to itself. Examples of magnetic materials include, but are not limited to ferrites, namely, iron oxides; rare earth metals; and other metals, for example, iron, nickel, and cobalt.

Generally, with friction-based binding technology, where friction arms are used to provide resistance needed to cause a binding plate to rotate, the plate is not allowed to rotate until an end-sensing arm of the plate reaches the end of a cannula. When the arm reaches the end of the cannula, the plate is rotated because it is being pushed to the distal end of the cannula only on one side of the binding plate. The friction arms are there to ensure that the plate rotates and "bites" into the cannula, locking the safety to the needle. Without the frictional arms, a safety device could slide off the end of the cannula if the plate does not rotate properly,

With the magnetic energy-based technology of the present invention, magnets are used to provide positive rotation in a binding component. The magnetic energy is used to push or pull the binding component off-center to ensure that it rotates and bites or binds a cannula. Typically, a magnetic energy safety device has no frictional arms present in the safety housing for storing mechanical energy, although it may be desirable to use a magnetic energy safety device in conjunction with friction-based binding. As an example, when the tip of a binding component having a front smooth tip reaches the end of the cannula, magnetic energy between, for example, the binding component and an area within a safety housing, causes the latch to rotate and bite on the cannula. The housing then presses against the binding component ensuring that it bites and locks on the cannula, thereby refraining the safety device from coming off of the needle tip. By reference to cannula, it is understood that cannulas of unlimited gauge sizes and needle tip geometries can be used. Also, safety devices of the present invention can be attached to many types of needle assemblies. Examples include, without limitation, short needles, long needles, needles attached to intravenous catheters, epidural needles, spinal needles, needles having luer adapters, syringes, and the like. In addition, a cannula having more than one diameter can be used in conjunction with embodiments of the present invention.

In some devices in accordance with the magnetic-energy technology of the present invention, the devices generally resist being retracted, re-exposing the needle tip. This is based on the forces of, for example, the magnet and housing combination pushing on the binding component on the opposite side during an attempted safety retraction. The binding component bites down into the cannula generally in the same orientation as when trying to slide it off the distal end. In examples where a transverse barrier is part of the binding component, the transverse barrier captures the needle tip and presents itself over the tip, not allowing the safety device to be retracted.

In one aspect of the present invention, safety needle assemblies are provided that comprise a cannula comprising a proximal end and a distal end; a hub disposed on the proximal end of the cannula; a housing partially surrounding a portion of the cannula, the housing comprising a distal end; and a magnetic latch that is operative against the cannula to secure the distal end of the cannula within the housing.

In some embodiments, the housing may have an open distal end. In this case, in some examples, a cover that partially surrounds a portion of the cannula can be associated with the housing in order to form an interior area. A cover, as desired, serves to close off the distal end of the housing. One example of a cover is a cap which is engagable with the housing. Another example of the cover is a portion of a can or sleeve that surrounds the exterior of the housing and closes off the distal end of the housing. The can or sleeve can be physically separate from the housing.

In an embodiment, the magnetic latch comprises a first magnetic material that is located in sufficient proximity to a second magnetic material to repel or attract the second magnetic material; and a binding component partially surrounding the cannula, the binding component comprising the second magnetic material.

In one embodiment, the assembly is free of a friction arm. By reference to free of a friction arm it is meant that a component used to store mechanical energy that has a close fit against the cannula is not present. Having a safety device that is free of friction arms eases assembly, for example, by reducing the number of parts that need to be handled or that are subject to damage during assembly. In addition, tactile response by users can also be improved.

The binding component can be made of any material, for example, metals or plastics. If the metal or plastic used does not have magnetic properties, then the binding component can be coated with magnetic material. In other cases, it may be desirable to use a separately fabricated magnetic material that is attached to the binding component.

In one embodiment, the second magnetic material is dispersed in a coating that is adhered to the binding component. In another embodiment, the second magnetic material is physically separate from the binding component and is attached to the binding component.

In some examples, the binding component further comprises an end sense member. In other examples, the binding component further comprises a transverse barrier. The second magnetic material can be located within or on the end sense member.

In one embodiment, the first magnetic material is attached to the cover. In another embodiment, the first magnetic material is dispersed in a coating that is adhered to a portion of the interior area. In yet another embodiment, the first magnetic material is attached to an interior surface of the housing. By interior surface of the housing, it is meant the surface of the housing that faces the cannula. In another embodiment, the first magnetic material is located on the exterior of the housing.

In certain examples, the first magnetic material and the second magnetic material attract each other. In other examples, the first magnetic material and the second magnetic material repel each other.

In one embodiment, the first magnetic material is attached to an interior surface of the housing, the binding component comprises an end sense member, and the first magnetic material attracts the second magnetic material. In another embodiment, the first magnetic material is attached to an interior surface of the housing, the binding component comprises an end sense member and a transverse barrier, and the first magnetic material attracts the second magnetic material.

In a further embodiment, the first magnetic material is attached to the cover; the binding component further comprises a transverse barrier; the second magnetic material is attached to the binding component; and the first magnetic material repels the second magnetic material.

In an embodiment, the first magnetic material is attached to the cover; the binding component comprises an end sense member; the second magnetic material is located within or on the end sense member; and the first magnetic material attracts the second magnetic material.

In one embodiment, the first magnetic material is attached to an interior surface of the housing; the binding component comprises an end sense member; the second magnetic material is located within or on the end sense member; and the first magnetic material repels the second magnetic material.

In a further embodiment, the first magnetic material comprises an electromagnet and a battery. In another embodiment, the safety needle assembly using an electromagnet and battery, further comprises a safety mechanism operatively associated with the housing having a first position that permits the electromagnet to be de-energized and a second position that permits the electromagnet to be energized. In one example, the safety mechanism comprises a switch.

In another aspect of the present invention, a method of making a safety needle assembly is provided, the method comprising: providing a cannula comprising a proximal end and a distal end; disposing a hub on the proximal end of the cannula; partially surrounding a portion of the cannula with a housing comprising a distal end; and locating a magnetic latch such that it is operative against the cannula.

In a further aspect of the present invention, a method of securing a needle tip is provided, the method comprising: moving a safety device from a proximal end of a cannula towards the needle tip located at a distal end, wherein the safety device comprises a housing and a magnetic latch; moving the end sense member past the needle tip toward the distal end of the cannula; and rotating the binding component with magnetic force, thereby securing the needle tip within the housing. In one example, the movement of the binding component along the cannula is substantially friction-free. By substantially friction-free it is meant that an aperture of the binding component is loose against the cannula. In this regard, an embodiment of the present invention can be substantially friction-free while using an end sense member because the aperture of the binding component can remain loose against the cannula. In terms of end sensing, it may be desirable to use features, for example, ferrule marks, bumps, notches, welded catches, and the like, on the cannula.

Before describing several exemplary embodiments of the invention, it is to be understood that the invention is not limited to the details of construction or process steps set forth in the following description. The invention is capable of other embodiments and of being practiced or being carried out in various ways.

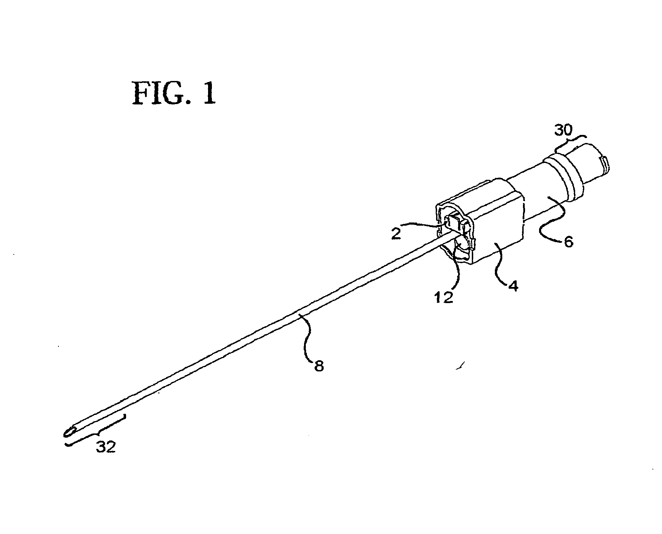

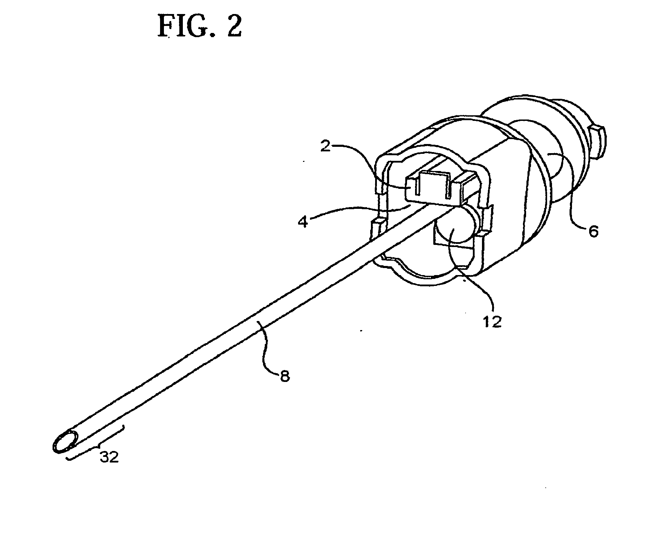

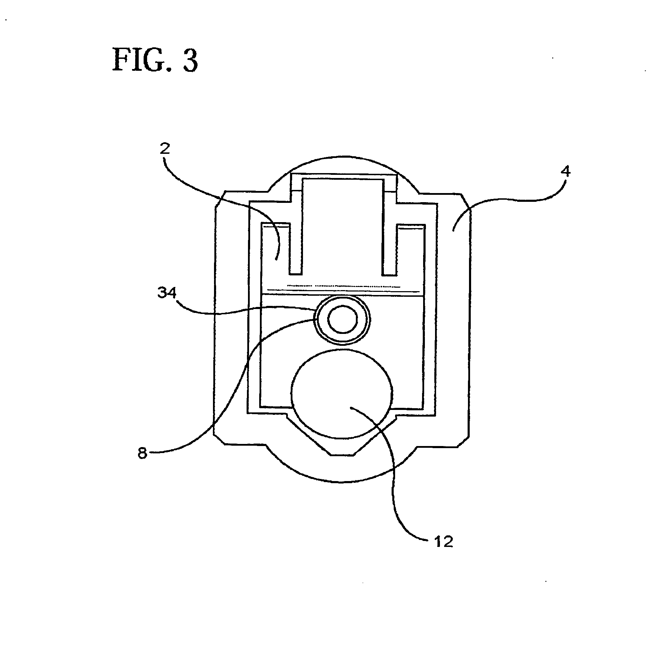

Turning to the figures, wherein like reference numerals refer to like elements, Figures 1 and 2 show needle assemblies and Figure 3 shows a cross-section view across a housing of a needle assembly. In one example, a magnetic latch comprises a binding component 2, also referred to as a binding plate, having magnetic properties and a magnet 12. The magnet 12, in this example, is located within the safety housing 4. In other examples, it may be desirable to locate a magnet outside of the safety housing. A hub 6 attaches to a proximal end 30 of a cannula 8 and partially surrounds the safety housing 4 and the cannula 8. In operation, the cannula 8 slides through an aperature 34.

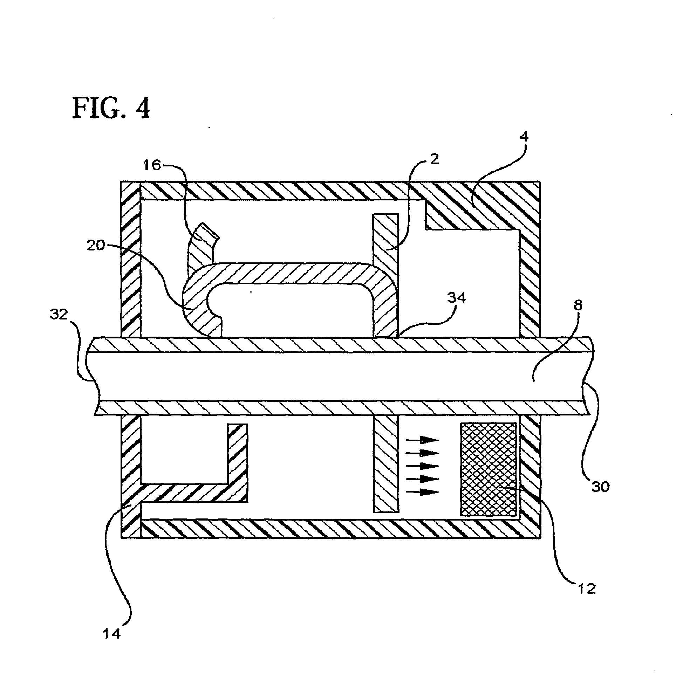

Figure 4 shows a schematic of the interior of the safety housing of an embodiment where the binding component 2 has an aperture 34, a transverse barrier 16, and an end sense member 20. In this example, the binding component 2 is made of material that is magnetically reactive. The safety cap 14 engages with the safety housing 4. A magnet 12 is located on the safety housing 4. The magnetically reactive binding component 2 is attracted to magnet 12. In practice, when the safety device is in position on the proximal end 30 of the cannula 8, the binding component 2 is substantially perpendicular to the longitudinal axis of the cannula 8, and therefore the edges of aperture 34 do not bite the cannula 8, due to the force of the end sense member 20 on the cannula 8. This is true even though the binding component 2 is attracted to magnet 12. When the safety device is moved from a first position at the proximal end 30 towards a second position at the distal end 32, the cannula 8 slides through the aperture 34. Once the needle tip is retracted into the safety device and the end sense member 20 clears the tip, the binding component 2 is no longer held in place by the end sense member 20 and the attracting magnetic force causes the binding component 2 to rotate and the edges of aperture 34 to bite down on the cannula 8. In this position, the transverse barrier 16 covers the tip.

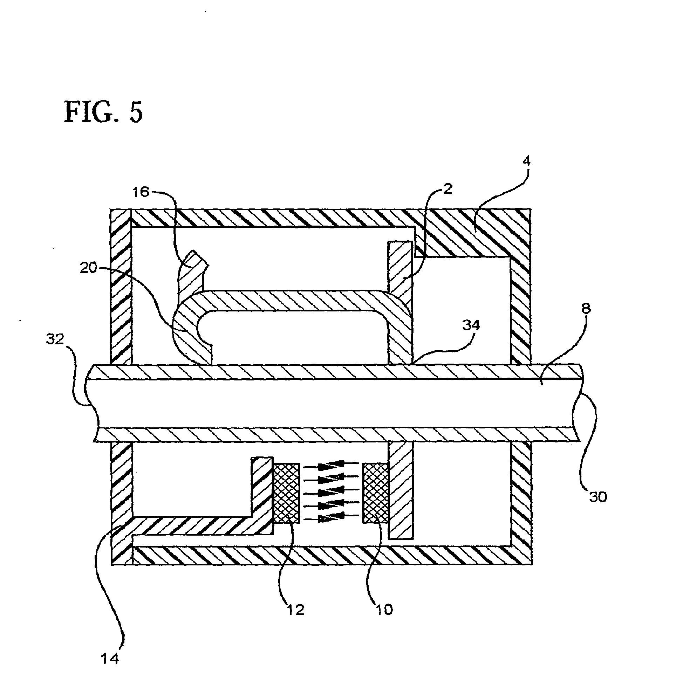

Figure 5 shows a schematic of the interior of the safety housing of an embodiment where a magnet 10 is attached to the binding component 2 having an aperture 34, a transverse barrier 16, and an end sense member 20. The safety cap 14 engages with the safety housing 4. In this example, the binding component 2 is made of material that is substantially magnetically un-reactive. A magnet 12 is attached to the safety cap 14. In this example, magnet 10 is repelled by magnet 12. In practice, when the safety device is in position on the proximal end 30 of the cannula 8, the binding component 2 is substantially perpendicular to the longitudinal axis of the cannula 8, and therefore the edges of aperture 34 do not bite the cannula 8, due to the force of the end sense member 20 on the cannula 8. This is true even though the magnet 10 located on the binding component 2 is repelled by magnet 12. When the safety device is moved from a first position at the proximale end 30 towards a second position at the distal end 32, the cannula 8 slides through the aperture 34. Once the needle tip is retracted into the safety device and the end sense member 20 clears the tip, the binding component 2 is no longer held in place by the end sense member 20 and the repelling magnetic force causes the binding component 2 to rotate and the edges of aperture 34 to bite down on the cannula 8. In this position, the transverse barrier 16 covers the tip.

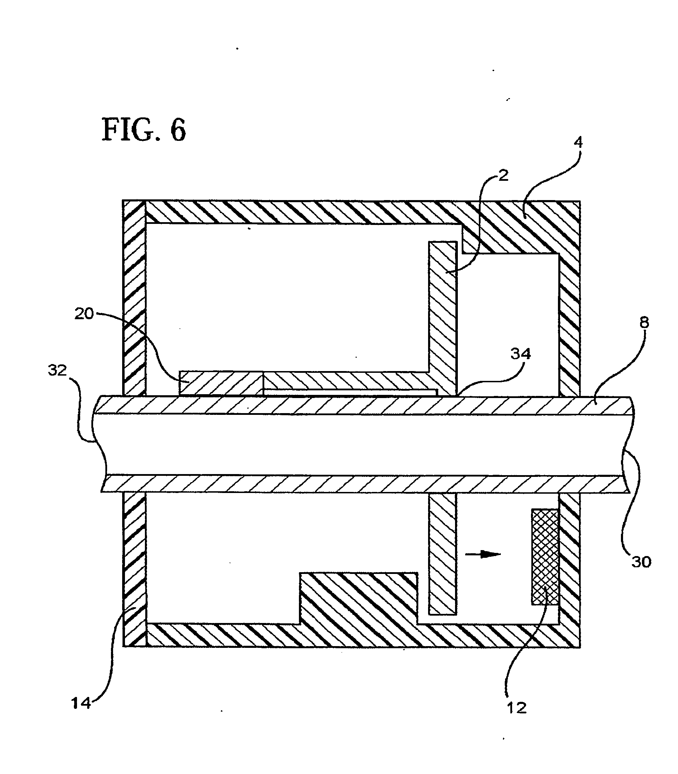

Figure 6 shows a schematic of the interior of the safety housing of an embodiment where the binding component 2 has an end sense member 20. The safety cap 14 engages with the safety housing 4. In this example, the binding component 2 is made of material that is magnetically reactive. A magnet 12 is located on the safety housing 4. In this example, the binding component 2 is attracted to magnet 12. In practice, when the safety device is in position on the proximal end 30 of the cannula 8, the binding component 2 is substantially perpendicular to the longitudinal axis of the cannula 8, and therefore the edges of aperture 34 do not bite the cannula 8, due to the force of the end sense member 20 on the cannula 8. This is true even though the binding component 2 is attracted to magnet 12. When the safety device is moved from a first position at the proximal end 30 towards a second position at the distal end 32, the cannula 8 slides through the aperture 34. Once the needle tip is retracted into the safety device and the end sense member 20 clears the tip, the binding component 2 is no longer held in place by the end sense member 20 and the attracting magnetic force causes the binding component 2 to rotate and the edges of aperture 34 to bite down on the cannula 8.

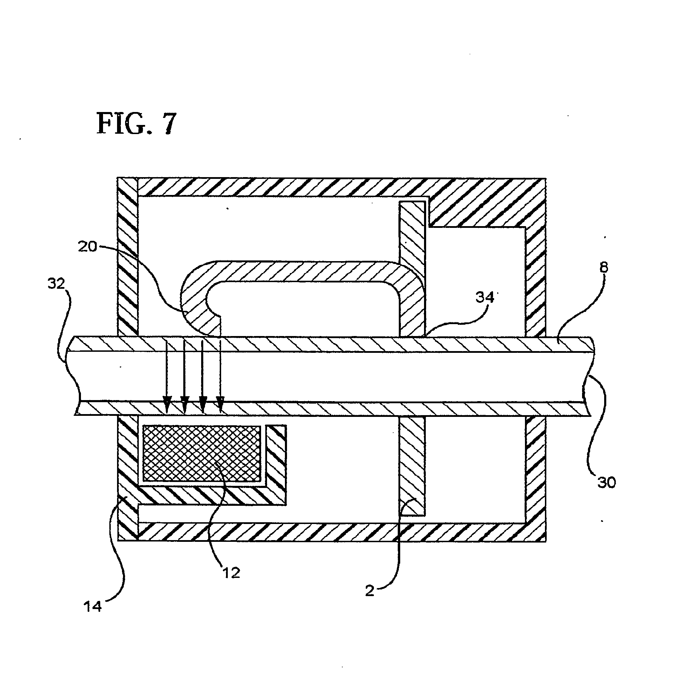

Figure 7 shows a schematic of the interior of the safety housing of an embodiment where the binding component 2 has an end sense member 20. In this example, the end sense member portion of the binding component is made of material that is magnetically reactive. The safety cap 14 engages with the safety housing 4. A magnet 12 is located on the safety cap 14. The magnetically reactive end sense member 20 is attracted to magnet 12. In practice, when the safety device is in position on the proximal end 30 of the cannula 8, the binding component 2 is substantially perpendicular to the longitudinal axis of the cannula 8, and therefore the edges of aperture 34 do not bite the cannula 8, due to the force of the end sense member 20 on the cannula 8. This is true even though the end sense member 20 of the binding component 2 is attracted to magnet 12. When the safety device is moved from a first position at the proximal end 30 towards a second position at the distal end 32, the cannula 8 slides through the aperture 34. Once the needle tip is retracted into the safety device and the end sense member 20 clears the tip, the binding component 2 is no longer held in place by the end sense member 20 and the attracting magnetic force causes the binding component 2 to rotate and the edges of aperture 34 to bite down on the cannula 8.

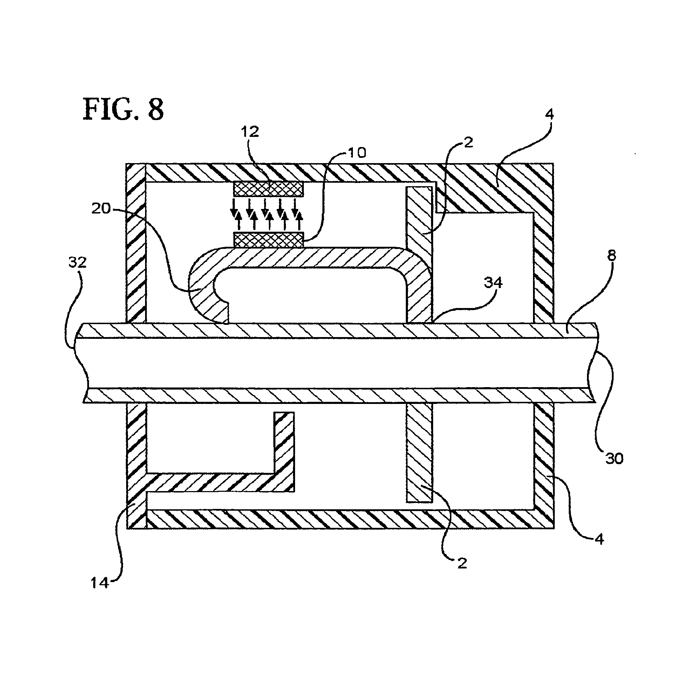

Figure 8 shows a schematic of the interior of the safety housing of an embodiment where a magnet 10 is attached to the end sense member 20 of the binding component 2. The safety cap 14 engages with the safety housing 4. In this example, the binding component 2 is made of material that is substantially magnetically un-reactive. A magnet 12 is attached to the safety housing 4. In this example, magnet 10 is repelled by magnet 12. In practice, when the safety device is in position on the proximal end 30 of the cannula 8, the binding component 2 is substantially perpendicular to the longitudinal axis of the cannula 8, and therefore the edges of aperture 34 do not bite the cannula 8, due to the force of the end sense member 20 on the cannula 8. This is true even though the magnet 10 on the binding component 2 is repelled by magnet 12. When the safety device is moved from a first position at the proximal end 30 towards a second position at the distal end 32, the cannula 8 slides through the aperture 34. Once the needle tip is retracted into the safety device and the end sense member 20 clears the tip, the binding component 2 is no longer held in place by the end sense member 20 and the repelling magnetic force causes the binding component 2 to rotate and the edges of aperture 34 to bite down on the cannula 8.

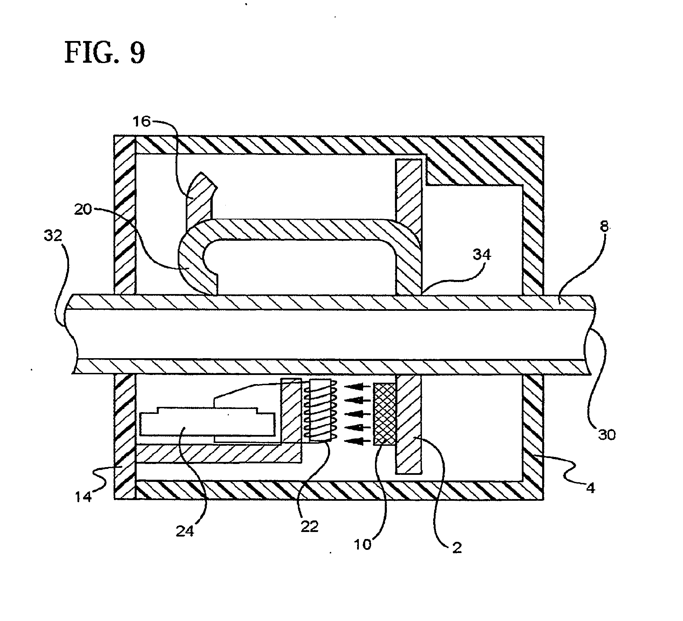

Figure 9 shows a schematic of the interior of the safety housing of an embodiment where a magnet 10 is attached to the binding component 2 having a transverse barrier 16 and an end sense member 20. The safety cap 14 engages with the safety housing 4. In this example, the binding component 2 is made of material that is substantially magnetically un-reactive. An electromagnet 22 is located on the safety cap 14. A battery 24 is connected to the electromagnet 22. In this example, magnet 10 is repelled by electromagnet 22. In practice, when the safety device is in position on the proximal end 30 of the cannula 8, the binding component 2 is substantially perpendicular to the longitudinal axis of the cannula 8, and therefore the edges of aperture 34 do not bite the cannula 8, due to the force of the end sense member 20 on the cannula 8. This is true even though the magnet 10 on the binding component 2 is repelled by magnet 12. When the safety device is moved from a first position at the proximal end 30 towards a second position at the distal end 32, the cannula 8 slides through the aperture 34. Once the needle tip is retracted into the safety device and the end sense member 20 clears the tip, the binding component 2 is no longer held in place by the end sense member 20 and the repelling magnetic force causes the binding component 2 to rotate and the edges of aperture 34 to bite down on the cannula 8. In this position, the transverse barrier 16 covers the tip.

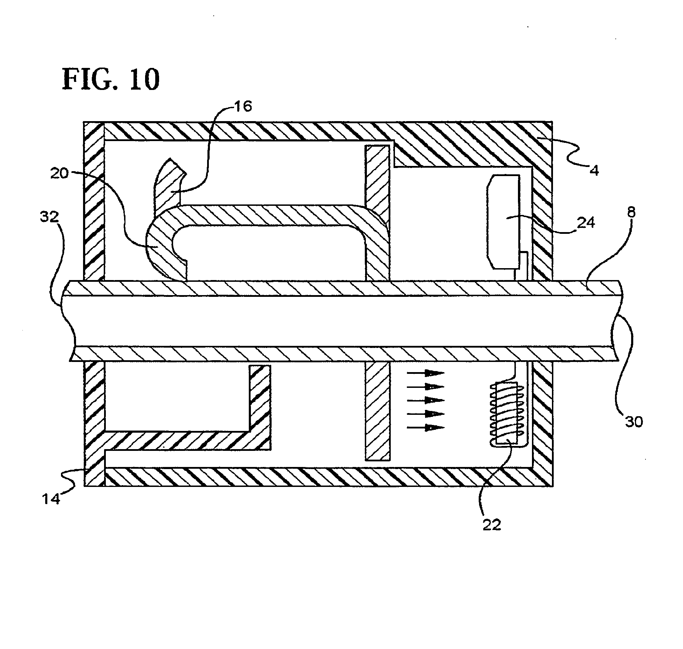

Figure 10 shows a schematic of the interior of the safety housing of an embodiment where the binding component 2 has a transverse barrier 16 and an end sense member 20. The safety cap 14 engages with the safety housing 4. In this example, the binding component 2 is made of material that is magnetically reactive. An electromagnet 22 is located on the safety housing 4. A battery 24 is connected to the electromagnet 22. In this example, the binding component 2 is attracted to the electromagnet 22. In practice, when the safety device is in position on the proximal end 30 of the cannula 8, the binding component 2 is substantially perpendicular to the longitudinal axis of the cannula 8, and therefore the edges of aperture 34 do not bite the cannula 8, due to the force of the end sense member 20 on the cannula 8. This is true even though the binding component 2 is attracted to electromagnet 22. When the safety device is moved from a first position at the proximal end 30 towards a second position at the distal end 32, the cannula 8 slides through the aperture 34. Once the needle tip is retracted into the safety device and the end sense member 20 clears the tip, the binding component is no longer held in place by the end sense member 20 and the attracting magnetic force causes the binding component 2 to rotate and the edges of aperture 34 to bite down on the cannula 8. In this position, the transverse barrier 16 covers the tip.

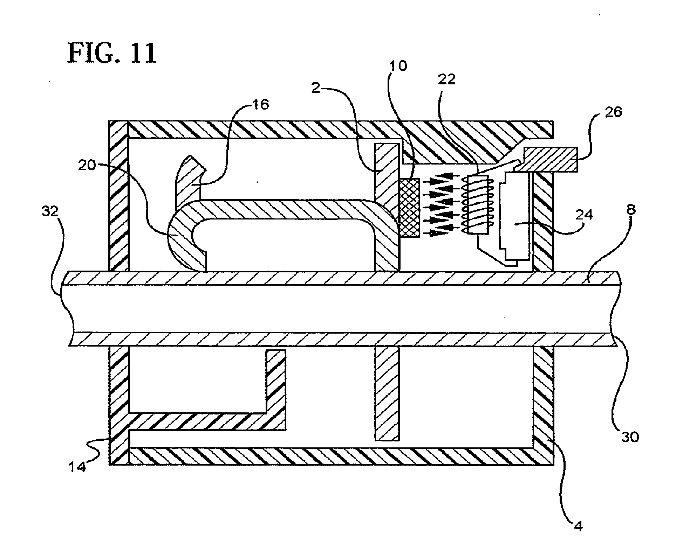

Figure 11 shows a schematic of the interior of the safety housing of an embodiment where the binding component 2 has a transverse barrier 16 and an end sense member 20. A magnet 10 is attached to the binding component 2. The safety cap 14 engages with the safety housing 4. In this example, the binding component 2 is made of material that is substantially magnetically un-reactive. An electromagnet 22 is attached to a battery 24 which is attached to the safety housing 4.

A safety mechanism comprising a switch 26 is provided in this example. In a first position, for example, when the safety device is located at the proximal end 30 of the cannula and the safety housing 4 is engaged with the hub (not shown), the switch 26 interrupts the electrical circuit between the battery 24 and the electromagnet 22. In a second position, the switch 26 is no longer in contact with the battery circuit, permitting the electrical circuit to be completed and causing the electromagnet to energize. The switch 26 can be in any form that is amenable to disrupting the electrical circuit between the battery and electromagnet. For example, the switch may be a piece integral to the needle assembly hub. In another example, it may be desirable that the switch is separate from the needle assembly hub. One example of this is a disposable piece of plastic that can be slid away from the safety housing in order to active the electromagnet. Prior to engaging the safety mechanism, the needle assembly of this embodiment is generally friction-free. In this example, magnet 10 is repelled by electromagnet 22.

In practice, when the safety device is in position on the proximal end 30 of the cannula 8, the binding component 2 is substantially perpendicular to the longitudinal axis of the cannula 8, and therefore the edges of aperture 34 do not bite the cannula 8, due to the force of the end sense member 20 on the cannula 8. At this point, there is no interaction between the magnet 10 on the binding component 2 and the electromagnet 22 because the electrical circuit is interrupted by switch 26. When the safety device is moved from a first position at the proximal end 30 towards a second position at the distal end 32, the cannula 8 slides through the aperture 34. Once the needle tip is retracted into the safety device and the end sense member 20 clears the tip, the binding component is no longer held in place by the end sense member. The electrical circuit engages when switch 26 is no longer interrupting the circuit. If the switch is an integral part of the hub, for example, then the movement of the safety housing 4 towards the distal end 32 causes the switch 26 to disengage from the circuit. If the switch is a separate piece of plastic, for example, then the circuit is completed upon removal of the plastic by, for example, a practitioner. Then, the repelling magnetic force causes the binding component 2 to rotate and the edges of aperture 34 to bite down on the cannula 8. In this position, the transverse barrier 16 covers the tip.

## Claims

1. A safety needle assembly comprising:
a cannula (8) comprising a proximal end and a distal end;
a hub (6) disposed on the proximal end of the cannula (8);
a safety housing (4) partially surrounding a portion of the cannula (8),
the housing (4) comprising a distal end;
a magnetic latch (10, 12, 22) that is operative against the cannula (8) to secure the distal end (32) of the cannula (8) within the housing (4);
and
a binding component (2), which rotates due to a magnetic force,
whereby edges of an aperture (34) of said binding component (2) bite down on or bind the cannula (8), when the needle tip (32) of the cannula (8) is retracted into the safety housing (4).

2. The safety needle assembly of claim 1 , wherein the magnetic latch comprises a first magnetic material that is located in sufficient proximity to a second magnetic material to repel or attract the second magnetic material; and the binding component (2) partially surrounding the cannula (8), the binding component comprising the second magnetic material.

3. The safety needle assembly of claim 2, wherein the first magnetic material is attached to an interior surface of the housing (4) and/or an exterior surface of the housing (4).

4. The safety needle assembly of claim 2, further comprising a cover (14) located at the distal end of the housing (4) and partially surrounds a portion of the cannula (8), wherein the first magnetic material is attached to the cover (14).

5. The safety needle assembly of claim 2, wherein the first magnetic material is dispersed in a coating that is adhered to a portion of an interior surface of the housing (4) and/or an exterior surface of the housing (4).

6. The safety needle assembly of claim 2, wherein the second magnetic material is dispersed in a coating that is adhered to a portion of the binding component (2) or is physically separate from the binding component (2) and is attached to the binding component (2).

7. The safety needle assembly of claim 6, further comprising a cover (14) located at the distal end of the housing (4) and partially surrounds a portion of the cannula (8), wherein the first magnetic material is attached to the cover (14).

8. The safety needle assembly of claim 6, wherein the first magnetic material is attached to an interior surface of the housing (4) and/or to an exterior surface of the housing (4).

9. The safety needle assembly of claim 2, wherein the first magnetic material and the second magnetic material attract each other and/or repel each other.

10. The safety needle assembly of claim 2, wherein the binding component (2) further comprises an end sense member, whereby preferably the second magnetic material is located within or on the end sense member.

11. The safety needle assembly of claim 3, wherein the binding component (2) further comprises an end sense member; and the first magnetic material attracts the second magnetic material and wherein the binding component (2) preferably further comprises a transverse barrier (16).

12. The safety needle assembly of claim 4, wherein the binding component (2) further comprises a transverse barrier (16); the second magnetic material is attached to the binding component (2); and the first magnetic material repels the second magnetic material or the binding component (2) further comprises an end sense member; the second magnetic material is located within or on the end sense member; and the first magnetic material attracts the second magnetic material.

13. The safety needle assembly of claim 3, wherein the binding component (2) further comprises an end sense member; the second magnetic material is located within or on the end sense member; and the first magnetic material repels the second magnetic material.

14. The safety needle assembly of claim 2, further comprising a safety mechanism operatively associated with the housing having a first position that permits the electromagnet to be de-energized and a second position that permits the electromagnet to be energized wherein the safety mechanism preferably comprises a switch (26).

15. The safety needle assembly of claim 2, wherein the first magnetic material is attached to an interior surface of the housing (4); the binding component (2) further comprises a transverse barrier (16); the second magnetic material is attached to the binding component (2); and the first magnetic material repels the second magnetic material.

16. A method of making a safety needle assembly comprising:
providing a cannula (8) comprising a proximal end and a distal end;
disposing a hub (6) on the proximal end of the cannula (8);
partially surrounding a portion of the cannula (8) with a safety housing (4) comprising a distal end; and
locating a magnetic latch (10, 12, 22) such that it is operative against the cannula (8) by a binding component (2) which rotates due to a magnetic force, whereby edges of the aperture (34) are binding components to bite down or bind the cannula (8), when the needle tip (32) of the cannula (8) is retracted into the safety housing (4).

17. A method of securing a needle tip comprising:
moving a safety device from a proximal end of a cannula (8) towards the needle tip located at a distal end (32), wherein the safety device comprises a housing (4) and a magnetic latch (10, 12, 22);
moving a portion of the magnetic latch (10, 12, 22) past the needle tip toward the distal end (32) of the cannula (8); and
rotating a binding component (32) with magnetic force, thereby securing the needle tip within the housing (4), whereby edges of an aperture (32) of that binding component (2) bite down on or bind the cannula (8).

18. The method of claim 17 wherein the portion of the magnetic latch (10, 12, 22) that moves past the needle tip comprises an end sense member of the binding component (2).

19. The method of claim 17 wherein the movement of the binding component (2) along the cannula (8) is substantially friction-free.

## Patentansprüche

1. Sicherheitsnadelanordnung mit:
einer Kanüle (8) mit einem proximalen Ende und einem distalen Ende;
einem an dem proximalen Ende der Kanüle (8) angeordneten Ansatz (6);
einem Sicherheitsgehäuse (4), welches einen Bereich der Kanüle (8) teilweise umgibt, wobei das Gehäuse (4) ein distales Ende aufweist;
einer Magnethalterung (10, 12, 22), die gegen die Kanüle (8) wirkt, um das distale Ende (32) der Kanüle (8) in dem Gehäuse (4) zu halten; und
einem Fixierelement (2), das aufgrund von Magnetkraft dreht,
wobei Ränder einer Öffnung (34) des Fixierelements (2) die Kanüle (8) festklemmen oder fixieren, wenn die Nadelspitze (32) der Kanüle (8) in das Sicherheitsgehäuse (4) zurückgezogen ist.

2. Sicherheitsnadelanordnung nach Anspruch 1, bei welcher die Magnethalterung ein erstes magnetisches Material aufweist, das in ausreichender Nähe zu einem zweiten magnetischen Material angeordnet ist, um das zweite magnetische Material abzustoßen oder anzuziehen; und bei welcher das Fixierelement (2) die Kanüle (8) teilweise umgibt, wobei das Fixierelement das zweite magnetische Material aufweist.

3. Sicherheitsnadelanordnung nach Anspruch 2, bei welcher das erste magnetische Material an einer Innenfläche des Gehäuses (4) und/oder einer Außenfläche des Gehäuses (4) angebracht ist.

4. Sicherheitsnadelanordnung nach Anspruch 2, ferner mit einer Abdeckung (14), die am distalen Ende des Gehäuses (4) angeordnet ist und einen Bereich der Kanüle (8) teilweise umgibt, wobei das erste magnetische Material an der Abdeckung (14) angebracht ist.

5. Sicherheitsnadelanordnung nach Anspruch 2, bei welcher das erste magnetische Material in einer Beschichtung dispergiert ist, welche an einem Bereich einer Innenfläche des Gehäuses (4) und/oder einer Außenseite des Gehäuses (4) haftend angebracht ist.

6. Sicherheitsnadelanordnung nach Anspruch 2, bei welcher das zweite magnetische Material in einer Beschichtung dispergiert ist, welche an einem Bereich des Fixierelements (2) haftend angebracht ist oder physisch von dem Fixierelement (2) getrennt und an dem Fixierelement (2) angebracht ist.

7. Sicherheitsnadelanordnung nach Anspruch 6, ferner mit einer Abdeckung (14), die am distalen Ende des Gehäuses (4) angeordnet ist und einen Bereich der Kanüle (8) teilweise umgibt, wobei das erste magnetische Material an der Abdeckung (14) angebracht ist.

8. Sicherheitsnadelanordnung nach Anspruch 6, bei welcher das erste magnetische Material an einer Innenfläche des Gehäuses (4) und/oder an einer Außenfläche des Gehäuses (4) angebracht ist.

9. Sicherheitsnadelanordnung nach Anspruch 2, bei welcher das erste magnetische Material und das zweite magnetische Material einander anziehen und/oder einander abstoßen.

10. Sicherheitsnadelanordnung nach Anspruch 2, bei welcher das Fixierelement (2) ferner ein Endfühlelement aufweist, wobei vorzugsweise das zweite magnetische Material in oder an dem Endfühlelement angeordnet ist.

11. Sicherheitsnadelanordnung nach Anspruch 3, bei welcher das Fixierelement (2) ferner ein Endfühlelement aufweist, und das erste magnetische Material das zweite magnetische Material anzieht, und wobei das Fixierelement (2) vorzugsweise ferner eine querverlaufende Sperre (16) aufweist.

12. Sicherheitsnadelanordnung nach Anspruch 4, bei welcher das Fixierelement (2) ferner eine querverlaufende Sperre (16) aufweist; das zweite magnetische Material an dem Fixierelement (2) angebracht ist; und das erste magnetische Material das zweite magnetische Material abstößt oder das Fixierelement (2) ferner ein Endfühlelement aufweist; das zweite magnetische Material in oder an dem Endfühlelement angeordnet ist; und das erste magnetische Material das zweite magnetische Material anzieht.

13. Sicherheitsnadelanordnung nach Anspruch 3, bei welcher das Fixierelement (2) ferner ein Endfühlelement aufweist; das zweite magnetische Material in oder an dem Endfühlelement angeordnet ist; und das erste magnetische Material das zweite magnetische Material abstößt.

14. Sicherheitsnadelanordnung nach Anspruch 2, ferner mit einem in Wirkverbindung mit dem Gehäuse stehenden Sicherheitsmechanismus, der eine erste Position, welche das Abschalten des Elektromagneten ermöglicht, und eine zweite Position aufweist, welche das Einschalten des Elektromagneten ermöglicht, wobei der Sicherheitsmechanismus vorzugsweise einen Schalter (26) aufweist.

15. Sicherheitsnadelanordnung nach Anspruch 2, bei welcher das erste magnetische Material an einer Innenfläche des Gehäuses (4) angebracht ist; das Fixierelement (2) ferner eine querverlaufende Sperre (16) aufweist; das zweite magnetische Material an dem Fixierelement (2) angebracht ist; und das erste magnetische Material das zweite magnetische Material abstößt.

16. Verfahren zur Herstellung einer Sicherheitsnadelanordnung mit den folgenden Schritten:
Vorsehen einer Kanüle (8) mit einem proximalen Ende und einem distalen Ende;
Anordnen eines Ansatzes (6) an dem proximalen Ende der Kanüle (8);
teilweises Umgeben eines Bereichs der Kanüle (8) mit einem Sicherheitsgehäuse (4), das ein distales Ende aufweist; und
Anordnen einer magnetischen Halterung (10, 12, 22) derart, dass sie mittels eines sich aufgrund einer Magnetkraft drehenden Fixierelements (2) gegen die Kanüle (8) wirkt, wobei Ränder der Öffnung (34) Fixierelemente sind, welche die Kanüle (8) festklemmen oder fixieren, wenn die Nadelspitze (32) der Kanüle (8) in das Sicherheitsgehäuse (4) zurückgezogen ist.

17. Verfahren zum Sichern einer Nadelspitze mit den folgenden Schritten:
Bewegen einer Sicherheitsvorrichtung von einem proximalen Ende einer Kanüle (8) in Richtung der an einem distalen Ende (32) befindlichen Nadelspitze, wobei die Sicherheitsvorrichtung ein Gehäuse (4) und eine magnetische Halterung (10, 12, 22) aufweist;
Bewegen eines Bereichs der magnetischen Halterung (10, 12, 22) über die Nadelspitze hinaus in Richtung des distalen Endes (32) der Kanüle (8);
und
Drehen eines Fixierelements (2) durch Magnetkraft, wodurch die Nadelspitze sicher in dem Gehäuse (4) gehalten wird, wobei die Ränder einer Öffnung (34) des Fixierelements (2) die Kanüle (8) festklemmen oder fixieren.

18. Verfahren nach Anspruch 17, bei welchem der Bereich der magnetischen Halterung (10, 12, 22), der über die Nadelspitze hinaus bewegt wird, ein Endfühlelement des Fixierelements (2) aufweist.

19. Verfahren nach Anspruch 17, bei welchem die Bewegung des Fixierelements (2) entlang der Kanüle (8) im Wesentlichen reibungsfrei ist.

## Revendications

1. Ensemble aiguille de sécurité comprenant:
une canule (8) ayant une extrémité proximale et une extrémité distale;
un embout (6) disposé à l'extrémité proximale de ladite canule (8);
un boitier de sécurité (4) partiellement entourant une partie de ladite canule (8), ledit boitier (4) comprenant une extrémité distale;
un dispositif de verrouillage magnétique (10, 12, 22) agissant contre ladite canule (8) pour fixer l'extrémité distale (32) de ladite canule (8) dans ledit boitier (4); et
un élément de serrage (2) qui tourne sous l'effet d'une force magnétique, des bords d'une ouverture (34) dudit élément de serrage (2) mordant ou serrant ladite canule (8) lorsque la pointe de l'aiguille (32) de ladite canule (8) est retirée dans le boitier de sécurité (4).

2. Ensemble aiguille de sécurité selon la revendication 1, dans lequel ledit dispositif de verrouillage magnétique comprend un premier matériau magnétique situé en proximité suffisante d'un deuxième matériau magnétique afin de repousser ou attirer le deuxième matériau magnétique; et ledit élément de fixation (2) entoure partiellement ladite canule (8), ledit élément de fixation comprenant ledit deuxième matériau magnétique.

3. Ensemble aiguille de sécurité selon la revendication 2, dans lequel ledit premier matériau magnétique est attaché à une surface intérieure du boitier (4) et/ou une surface extérieure du boitier (4).

4. Ensemble aiguille de sécurité selon la revendication 2, comprenant en outre un couvercle (14) situé à l'extrémité distale dudit boitier (4) et partiellement entourant une partie de la canule (8), ledit premier matériau magnétique étant attaché au couvercle (14).

5. Ensemble aiguille de sécurité selon la revendication 2, dans lequel le deuxième matériau magnétique est dispersé dans un revêtement qui adhère à une partie d'une surface intérieure dudit boitier (4) et/ou une surface extérieure dudit boitier (4).

6. Ensemble aiguille de sécurité selon la revendication 2, dans lequel le deuxième matériau magnétique est dispersé dans un revêtement qui adhère à une partie dudit élément de serrage (2) ou est physiquement séparé dudit élément de serrage (2) et est attaché audit élément de serrage (2).

7. Ensemble aiguille de sécurité selon la revendication 6, comprenant en outre un couvercle (14) situé à l'extrémité distale dudit boitier (4) et partiellement entourant une partie de la canule (8), ledit premier matériau magnétique étant attaché au couvercle (14).

8. Ensemble aiguille de sécurité selon la revendication 6, dans lequel ledit premier matériau magnétique est attaché à une surface dudit boitier (4) et/ou à une surface extérieure dudit boitier (4).

9. Ensemble aiguille de sécurité selon la revendication 2, dans lequel ledit premier matériau magnétique et le deuxième matériau magnétique s'attirent et/ou se repoussent.

10. Ensemble aiguille de sécurité selon la revendication 2, dans lequel ledit élément de serrage (2) en outre comprend un élément capteur de fin de course, ledit deuxième matériau magnétique, de préférence, étant situé dans ou sur ledit élément capteur de fin de course.

11. Ensemble aiguille de sécurité selon la revendication 3, dans lequel ledit élément de serrage (2) en outre comprend un élément capteur de fin de course; et ledit premier matériau magnétique attire ledit deuxième matériau magnétique, et ledit élément de serrage (2) préférentiellement comprend en outre une barrière transversale (16).

12. Ensemble aiguille de sécurité selon la revendication 4, dans lequel ledit élément de serrage (2) en outre comprend une barrière transversale (16); ledit deuxième matériau magnétique est attaché audit élément de serrage (2); et ledit premier matériau magnétique repousse le deuxième matériau magnétique, ou ledit élément de serrage (2) en outre comprend un élément capteur de fin de course; ledit deuxième matériau magnétique est situé dans ou sur ledit élément capteur de fin de course; et ledit premier matériau magnétique attire le deuxième matériau magnétique.

13. Ensemble aiguille de sécurité selon la revendication 3, dans lequel ledit élément de serrage (2) comprend en outre un élément capteur de fin de course; ledit deuxième matériau magnétique est situé dans ou sur ledit élément capteur de fin de course; et ledit premier matériau magnétique repousse le deuxième matériau magnétique.

14. Ensemble aiguille de sécurité selon la revendication 2, comprenant en outre un mécanisme de sécurité associé en fonctionnement audit boitier et comprenant une première position permettant la désactivation de l'électroaimant et une deuxième position permettant l'activation dudit électroaimant, ledit mécanisme de sécurité préférentiellement comprenant un interrupteur (26).

15. Ensemble aiguille de sécurité selon la revendication 2, dans lequel ledit premier matériau magnétique est attaché à une surface intérieure du boitier (4); ledit élément de serrage (2) en outre comprend une barrière transversale (16); le deuxième matériau magnétique est attaché audit élément de serrage (2); et ledit premier matériau magnétique repousse ledit deuxième matériau magnétique.

16. Procédé de fabrication d'un ensemble aiguille de sécurité comprenant les étapes suivantes:
prévoir une canule (8) comprenant une extrémité proximale et une extrémité distale;
disposer un embout (6) à l'extrémité distale de ladite canule (8);
entourer partiellement une partie de la canule (8) avec un boitier de sécurité (4) ayant une extrémité distale; et
disposer un dispositif de verrouillage magnétique (10, 12, 22) de sorte que celui-ci agit contre la canule (8) au moyen d'un élément de fixation (2) qui tourne à cause d'une force magnétique, des bords d'une ouverture (34) formant des éléments de serrage mordant ou serrant ladite canule (8) lorsque la pointe de l'aiguille (32) de ladite canule (8) est retirée dans ledit boitier de sécurité (4).

17. Procédé de protection d'une pointe d'aiguille comprenant les étapes suivantes:
déplacer un dispositif de sécurité à partir d'une extrémité proximale d'une canule (8) vers la pointe d'aiguille située à une extrémité distale (32), ledit dispositif de sécurité comprenant un boitier (4) et un dispositif de verrouillage magnétique (10, 12, 22);
déplacer une partie dudit dispositif de verrouillage magnétique (10, 12, 22) au-delà de la pointe d'aiguille vers l'extrémité distale (32) de ladite canule (8); et tourner un élément de serrage (2) par force magnétique, ainsi retenant la pointe d'aiguille de manière protégée dans le boitier (4), des bords d'une ouverture (34) dudit élément de serrage (2) mordant ou serrant ladite canule (8).

18. Procédé selon la revendication 17, dans lequel la partie dudit dispositif de verrouillage magnétique (10, 12, 22) qui est déplacée au-delà de la pointe d'aiguille comprend un élément capteur de fin de course dudit élément de serrage (2).

19. Procédé selon la revendication 17, dans lequel le déplacement dudit élément de serrage (2) le long de la canule (8) est sensiblement sans friction.
